# EUROPEAN PATENT APPLICATION

(11) **EP 4 268 875 A1**
(43) Date of publication of application: **01.11.2023**
(21) Application number: 21909993.4
(22) Date of filing: 08.11.2021
(51) Int. Cl.: A61M 25/00, A61N 1/06, A61B 18/12, A61B 17/32

(54) **CATHETER**

(30) Priority: 25.12.2020 JP 2020216725
(71) Applicant: Asahi Intecc Co., Ltd., Seto-shi, Aichi 489-0071 (JP)
(72) Inventor: KAWAMURA, Kenta, Seto-shi, Aichi 489-0071 (JP); MIHARA, Shota, Seto-shi, Aichi 489-0071 (JP); SUGAWARA, Kei, Seto-shi, Aichi 489-0071 (JP); SAKATA, Kensuke, Seto-shi, Aichi 489-0071 (JP)
(74) Representative: Winter, Brandl - Partnerschaft mbB
(86) International application number: PCT/JP2021/040914
(87) International publication number: WO 2022/137839

(57) **Abstract**

A catheter according to the present invention is provided with: a hollow shaft; a distal end electrode provided on a distal end portion of the hollow shaft; a proximal end electrode provided on a proximal end portion of the hollow shaft, and which is electrically connected to the distal end electrode via the hollow shaft; and a connection portion which is capable of being attached to and detached from the proximal end electrode and which, by being attached to the proximal end electrode, electrically connects an external voltage output device and the proximal end electrode via a lead wire.

## Description

### TECHNICAL FIELD

The disclosed embodiments relate to a catheter.

### BACKGROUND ART

In recent years, minimally invasive catheter ablation has been performed as a representative treatment method for arrhythmia and the like. In catheter ablation, a medium or high frequency current is applied from the distal end of a catheter to the vicinity of a lesion to cauterize the lesion.

Patent Literature 1 discloses an energization system for a cardiac ablation catheter. In the catheter system described in Patent Literature 1, an attachment having a luer lock mechanism is provided on a hand-held portion of the catheter, and a lead wire and a connection terminal are provided for electrical connection to an output device via the attachment.

Patent Literature 2 discloses an electrode catheter used in examination (diagnosis) and treatment of arrhythmia and the like. This electrode catheter includes an electrode provided at the distal end of a catheter tube, and a conductive wire connected to a connector provided on a handle provided at the near end of the catheter tube, and a portion of the handle is split and is detachably formed. When the electrode catheter is manufactured, the electrical connection is made by detaching the split piece and soldering the proximal end of the conductive wire to the connector.

### CITATION LIST

### Patent Literature

Patent Literature 1: JP H8-508917 W
Patent Literature 2: JP 2014-180498 A

### SUMMARY OF INVENTION

### TECHNICAL PROBLEM

In catheter treatment such as PCI (percutaneous coronary intervention), an operator is required to perform delicate operations so as to not cause vascular perforation or intimal damage. However, when the catheter described in Patent Literature 1 is used, operation is difficult because of the attachment and the lead wire that are provided. In particular, when performing a rotation operation of the catheter, the lead wire may become entangled with the shaft, which can hinder operation. In the electrode catheter described in Patent Literature 2, no consideration has been given to the reduction in operability caused by connecting the electrode catheter to an external device via the lead wire. Such problems are common to all catheters inserted into biological lumens such as the blood vascular system, the lymph gland system, bile tract system, urinary system, respiratory tract system, digestive organ system, secreting gland system, and reproductive organs.

The disclosed embodiments have been made to solve at least a part of the above problems and have an object of providing, in a catheter including an electrode, a technique that improves the operability of the catheter.

### SOLUTION TO PROBLEM

The disclosed embodiments have been made in order to solve at least a part of the above problems, and can be implemented as the following aspects.
(1) According to an aspect of the disclosed embodiments, a catheter is provided. The catheter includes: a hollow shaft; a distal end electrode provided on a distal end portion of the hollow shaft; a proximal end electrode provided on a proximal end portion of the hollow shaft, and which is electrically connected to the distal end electrode via the hollow shaft; and a connection portion which is capable of being attached to and detached from the proximal end electrode and which, by being attached to the proximal end electrode, electrically connects an external voltage output device and the proximal end electrode via a lead wire.
   According to this configuration, a connection portion which is capable of being attached to and detached from the proximal end electrode is provided, and therefore, when an operator inserts the catheter into the body and performs an operation to advance the catheter inside a blood vessel, the connection portion can be detached from the proximal end electrode. As a result of detaching the connection portion, the same operability as a catheter not having a lead wire and an electrode can be obtained. On the other hand, when the distal end electrode of the catheter reaches the target position and conduction becomes necessary to perform cauterization or the like, energization can be established by attaching the connection portion to the proximal end electrode and connecting to an external voltage output device. That is, as a result of providing the connection portion that can be attached and detached, good operability is obtained when conduction is not necessary, and a catheter that can be energized when necessary can be provided.
(2) In the catheter according to the aspect above, the connection portion may be provided with a covering portion that covers the proximal end electrode when attached to the proximal end electrode. As a result, because the proximal end electrode is covered by the connection portion, the possibility of electrical leakage due to exposure of the proximal end electrode can be reduced.
(3) The catheter according to the aspects above may further include a grip portion provided on a proximal end portion of the hollow shaft, the grip portion being provided with a distal end grip portion disposed on a distal end portion of the grip portion, a proximal end grip portion disposed on a proximal end portion of the grip portion, and an intermediate grip portion disposed between the distal end grip portion and the proximal end grip portion; and the connection portion may configure the intermediate grip portion. As a result, because the connection portion also functions as the intermediate grip portion, good operability of the catheter can be obtained even when the connection portion is attached.
(4) The catheter according to the aspects above may further include a replacement portion that is capable of being attached to and detached from the proximal end electrode, and when the connection portion is not attached to the proximal end electrode and the replacement portion is attached to the proximal end electrode instead of the connection portion, configures the intermediate grip portion that does not electrically connect the proximal end electrode and the voltage output device. As a result, because the proximal end electrode is covered by the replacement portion even when the connection portion is not attached, the possibility of electrical leakage can be reduced.
(5) In the catheter according to the aspects above, the covering portion may include a first cover portion having a first connection terminal that is connected to the lead wire, and a second cover portion joined with the first cover portion by a joint portion having a hinge structure, and may be configured to be capable of opening and closing, attached to the proximal end electrode in a closed state in which the proximal end electrode is held between the first cover portion and the second cover portion, and capable of being detached from the proximal end electrode in an open state, and the first connection terminal may be disposed in a position making contact with the proximal end electrode when the connection portion is attached to the proximal end electrode. As a result, the connection portion can be more easily attached and detached by opening and closing operations. Consequently, conduction can be rapidly provided to the catheter. Furthermore, when the connection portion is attached to the proximal end electrode, because the first connection terminal is disposed in a position making contact with the proximal end electrode, conduction can be easily ensured by attaching the connection portion by the simple operation of setting the first cover portion and the second cover portion to the closed state.
(6) In the catheter according to the aspects above, at least one of the first cover portion and the second cover portion is provided with a groove portion for an electrode in which the proximal end electrode can be fitted, and the groove portion for an electrode is disposed in a position in which the first connection terminal makes contact with the proximal end electrode when the connection portion is attached to the proximal end electrode. As a result, the proximal end electrode and the first connection terminal are easily disposed in appropriate positions, which enables an electrical connection with an external voltage output device to be made with high accuracy.
(7) In the catheter according to the aspects above, at least one of the first cover portion and the second cover portion may be provided with a seal portion that surrounds an outer periphery of the groove portion for an electrode. As a result, entry of water into the proximal end electrode can be suppressed, which enables electrical leakage to be suppressed.
(8) In the catheter according to the aspects above, the proximal end grip portion may be provided with a support portion that is provided on a distal end side of the proximal end grip portion and fixedly supports the proximal end electrode, the support portion may have a protrusion portion that protrudes from a central axis of the hollow shaft toward a direction of an outer periphery of the grip portion, the intermediate grip portion may be provided with a concave portion that is fitted with the support portion of the proximal end grip portion. As a result, when an operator grips the intermediate grip portion and performs a rotation operation of the catheter, because the rotational force applied to the intermediate grip portion is transmitted to the proximal end electrode via the support portion, idle rotation of the intermediate grip portion is suppressed, and the torquability of the catheter can be improved.
(9) In the catheter according to the aspects above, the distal end grip portion may be provided with a tapered portion having a diameter that decreases toward its own distal end, and the tapered portion may have, on a surface, a spiral groove portion formed having a spiral groove shape. As a result, the tapered portion is made flexible, and kinking of the hollow shaft can be suppressed.
(10) In the catheter according to the aspect above, the distal end grip portion may be capable of supporting the lead wire along a surface of the distal end grip portion when the intermediate grip portion is configured by the connection portion, and may have, on its own surface, a concave portion for a lead wire in which a lead wire can be fitted. As a result, because the lead wire can be aligned with the surface of the distal end grip portion, it is possible to suppress a reduction in operability caused by the lead wire.
(11) In the catheter according to the aspects above, the lead wire may be provided with a second connection terminal that is disposed on its own distal end and is capable of being electrically connected to the voltage output device, and the catheter may be provided with a terminal support portion on the distal end grip portion that is capable of supporting the second connection terminal. As a result, when the electrical connection between the second connection terminal and the voltage output device is disconnected and the catheter is operated in a state where the connection portion is attached, the second connection terminal can be fixed to the distal end grip portion by the terminal support portion. Consequently, the second connection terminal of the lead wire can move freely, which can suppress a reduction in operability caused by the lead wire becoming entangled with the catheter and the like.

The disclosed embodiments can be implemented in various forms, and can be implemented, for example, in the form of a catheter or a catheter system including a catheter, a sensor, and other devices such as a delivery guide wire, a plasma guide wire, and the like.

### BRIEF DESCRIPTION OF DRAWINGS

Fig. 1 is a schematic diagram of the overall configuration of a plasma guide wire system according to a first embodiment.
Fig. 2 is an explanatory diagram showing a schematic configuration of a plasma catheter according to the first embodiment.
Fig. 3 is an explanatory diagram of a configuration of a grip portion of a plasma catheter.
Fig. 4 is an explanatory diagram schematically showing a configuration of a hollow shaft and a proximal end electrode.
Fig. 5 is an explanatory diagram schematically showing a configuration of a support portion that fixedly supports a proximal end electrode.
Fig. 6 is an explanatory diagram schematically showing a configuration of a connection portion.
Fig. 7 is an explanatory diagram schematically showing inner surface configurations of a first cover portion and a second cover portion.
Fig. 8 is an explanatory diagram schematically showing a distal end surface of a connection portion.
Fig. 9 is an explanatory diagram schematically showing a proximal end surface of a connection portion.
Fig. 10 is an explanatory diagram showing how a first connection terminal makes contact with a proximal end electrode.
Fig. 11 is an explanatory diagram schematically showing the appearance of a distal end grip portion of a plasma catheter according to a second embodiment.
Fig. 12 is an explanatory diagram schematically showing the appearance of a terminal support portion of a plasma catheter according to a third embodiment.
Fig. 13 is an explanatory diagram showing a schematic configuration of a plasma catheter according to a fourth embodiment.
Fig. 14 is an explanatory diagram schematically showing a configuration of a support portion that fixedly supports a proximal end electrode.
Fig. 15 is an explanatory diagram schematically showing a proximal end surface of a connection portion.
Fig. 16 is an explanatory diagram schematically showing the external configuration of a plasma catheter according to a fifth embodiment.

### EMBODIMENTS OF THE INVENTION

### <First Embodiment>

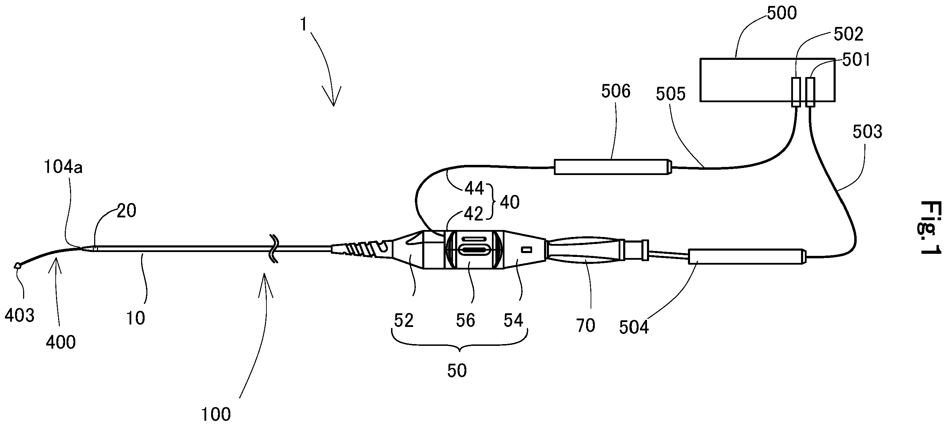

Fig. 1 is a schematic diagram of the overall configuration of a plasma guide wire system. A plasma guide wire system is mainly used when treating CTO (Chronic Total Occlusion) with an antegrade approach. In addition to CTO treatment, a plasma guide wire system may be used to treat mild to moderate stenosis, significant stenosis, arrhythmia, and the like. The plasma guide wire system 1 is configured by a plasma catheter 100, a plasma guide wire 400, and an RF generator 500. Fig. 1 shows a schematic side view of the plasma catheter 100. For convenience of the description, Fig. 1 includes sections where the components are illustrated with a relative size ratio which is different from the actual relative ratio. Furthermore, Fig. 1 includes sections where the illustration of portions of the components are exaggerated. In Fig. 1, the left side is referred to as the "distal end side" of the components, and the right side is referred to as the "proximal end side" of the components. Moreover, in each component, the end portion located on the distal end side is referred to as the "distal end", and the end portion located on the proximal end side is referred to as the "proximal end". In addition, a section located on or near the distal end is referred to as the "distal end portion", and a section located on or near the proximal end is referred to as the "proximal end portion". The distal end side is inserted into a living body, and the proximal end side is operated by an operator such as a surgeon. These points are also true for the drawings following Fig. 1.

The plasma catheter 100 includes a hollow shaft 10, a distal end electrode 20 provided on a distal end portion of the hollow shaft 10, a proximal end electrode (described later) provided on a proximal end portion of the hollow shaft 10, a grip portion 50 provided on the proximal end portion of the hollow shaft 10, and a connector 70. The plasma guide wire 400 is provided with a distal tip 403 on the distal end. The distal tip 403 is made of a conductive metallic material, such as chromium molybdenum steel, nickel chromium molybdenum steel, stainless steel such as SUS304, nickel titanium alloy, or the like.

The RF generator 500 outputs high frequency power between a terminal 501 and a terminal 502. The terminal 501 is connected to the plasma guide wire 400 via a cable 503 and a cable connector 504. The terminal 502 is connected to the plasma catheter 100 via a cable 505 and a cable connector 506.

As illustrated, in the plasma guide wire system 1, the plasma guide wire 400 is inserted into the plasma catheter 100, and is used as a result of the distal end portion being disposed so as to protrude from the distal end of the plasma catheter 100.

When the plasma catheter 100 is delivered to the CTO and the distal end portion of the plasma guide wire 400 protrudes from the distal end of the plasma catheter 100, and high frequency power is output from the RF generator 500 between the terminal 501 and the terminal 502, the voltage difference between the distal end electrode 20 of the plasma catheter 100 and the distal tip 403 of the plasma guide wire 400 causes a streamer corona discharge to be generated at the distal tip 403. The streamer corona discharge can ablate the CTO.

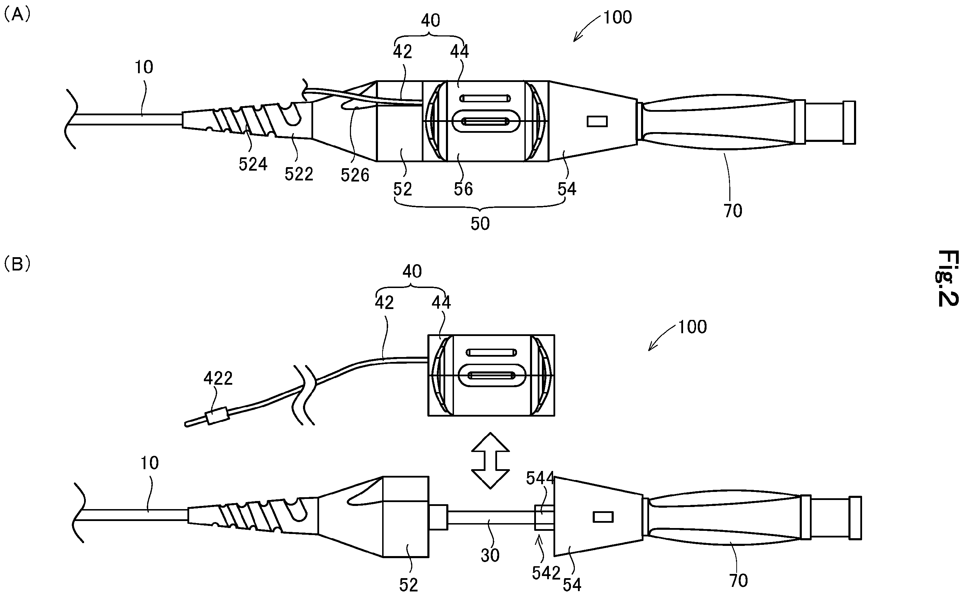

Fig. 2 is an explanatory diagram showing a schematic configuration of the plasma catheter 100 according to the first embodiment. As mentioned above, the plasma catheter 100 includes the grip portion 50 on the proximal end portion of the hollow shaft 10. As shown in Fig. 2(A), the grip portion 50 includes a distal end grip portion 52 disposed on the distal end portion of the grip portion 50, a proximal end grip portion 54 disposed on the proximal end portion of the grip portion 50, and an intermediate grip portion 56 disposed between the distal end grip portion 52 and the proximal end grip portion 54. The grip portion 50 is formed of an insulating resin.

The intermediate grip portion 56 may be configured by a connection portion 40 that can be electrically connected the RF generator 500 via a lead wire 42. As shown in Fig. 2(B), the proximal end portion of the hollow shaft 10 is provided with a proximal end electrode 30, which is electrically connected to the distal end electrode 20 (Fig. 1) via the hollow shaft 10. The connection portion 40 is formed so as to be capable of being attached to and detached from the proximal end electrode 30. Fig. 2(A) shows a state where the connection portion 40 is attached to the proximal end electrode 30, and Fig. 2(B) shows a state where the connection portion 40 has been detached from the proximal end electrode 30.

The connection portion 40 includes a covering portion 44 that covers the proximal end electrode 30 when attached to the proximal end electrode 30, and a lead wire 42 (Fig. 2(A)). The lead wire 42 includes, on its own distal end, a second connection terminal 422 that can be electrically connected to the RF generator 500. In the present embodiment, the second connection terminal 422 is connected to the cable connector 506, and is electrically connected to the RF generator 500 via the cable connector 506 (Fig. 1), the cable 505, and the terminal 502. The covering portion 44 includes, on the inside, a first connection terminal (described in detail later) that connects to the proximal end electrode 30, and the proximal end electrode 30 and the RF generator 500 are electrically connected when the connection portion 40 is attached to the proximal end electrode 30, and the second connection terminal 422 is connected to the RF generator 500 via the cable connector 506, the cable 505, and the terminal 502.

The distal end grip portion 52 includes a tapered portion 522 having a diameter that decreases toward its own distal end. The tapered portion 522 has, on the surface, a spiral groove portion 524 formed having a spiral groove shape. Because the distal end grip portion 52 has the tapered portion 522 formed with the spiral groove portion 524, sudden changes in the rigidity between the distal end grip portion 52 and the hollow shaft 10 can be reduced, and a gradual change in the rigidity between the distal end grip portion 52 and the hollow shaft 10 can be achieved. As a result, kinking of the hollow shaft 10 can be suppressed. Furthermore, when the intermediate grip portion 56 is configured by the connection portion 40, the distal end grip portion 52 is capable of supporting the lead wire 42 along the surface, and the distal end grip portion 52 has, on its own surface, a concave portion 526 for a lead wire to which the lead wire can be fitted. Because the distal end grip portion 52 includes the concave portion 526 for a lead wire, the lead wire 42 can be aligned with the surface of the distal end grip portion, and it is possible to suppress a reduction in operability caused by the lead wire 42.

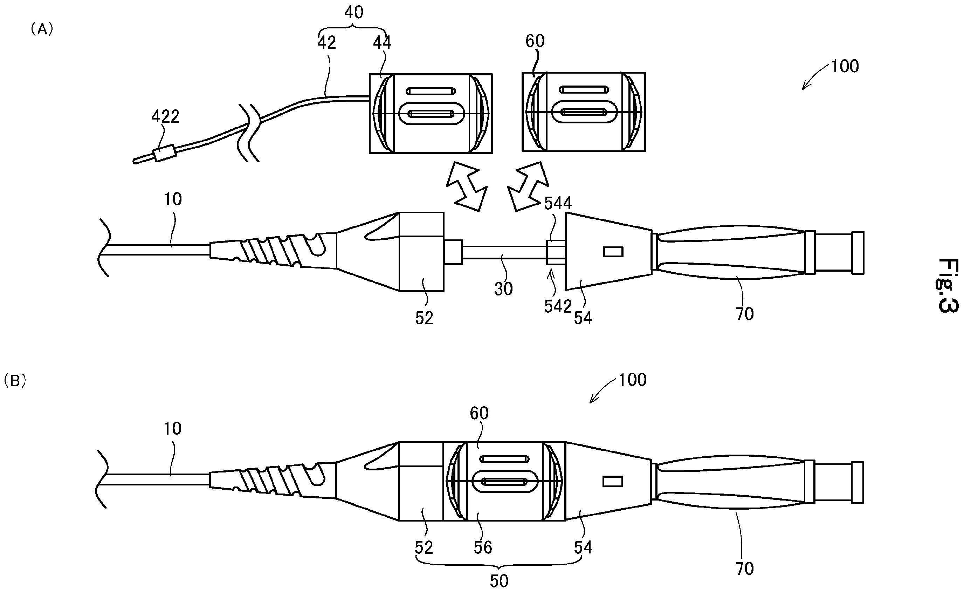

Fig. 3 is an explanatory diagram of a configuration of the grip portion 50 of the plasma catheter 100. As mentioned above, in the plasma catheter 100 according to the present embodiment, the intermediate grip portion 56 can be configured by the connection portion 40. Furthermore, the intermediate grip portion 56 may be configured by a replacement portion 60. That is, the plasma catheter 100 is configured such that the connection portion 40 and the replacement portion 60 can be exchanged (Fig. 3(A)). Like the connection portion 40, the replacement portion 60 is capable of being attached to and detached from the proximal end electrode 30, and configures the intermediate grip portion 56 by being attached to the proximal end electrode 30 instead of the connection portion 40 when the connection portion 40 is not attached to the proximal end electrode 30 (Fig. 3(B)). The replacement portion 60 does not include the lead wire 42, and also does not include a connection terminal that connects to the proximal end electrode 30, and therefore, when the replacement portion 60 is attached to the proximal end electrode 30, the proximal end electrode 30 and the RF generator 500 are not electrically connected. When the replacement portion 60 is attached to the proximal end electrode 30 and configures the intermediate grip portion 56, a reduction in operability caused by the lead wire becoming entangled with the grip portion 50 and the hollow shaft 10 can be suppressed. Furthermore, the replacement portion 60 does not include the lead wire 42, and also does not include a connection terminal that connects to the proximal end electrode 30, and therefore, it is lighter than the connection portion 40 and operation is easier compared to a case where the connection portion 40 is attached. Consequently, when the replacement portion 60 is attached, the plasma catheter 100 can exhibit the same operability as that of a typical catheter that does not have an energizing configuration.

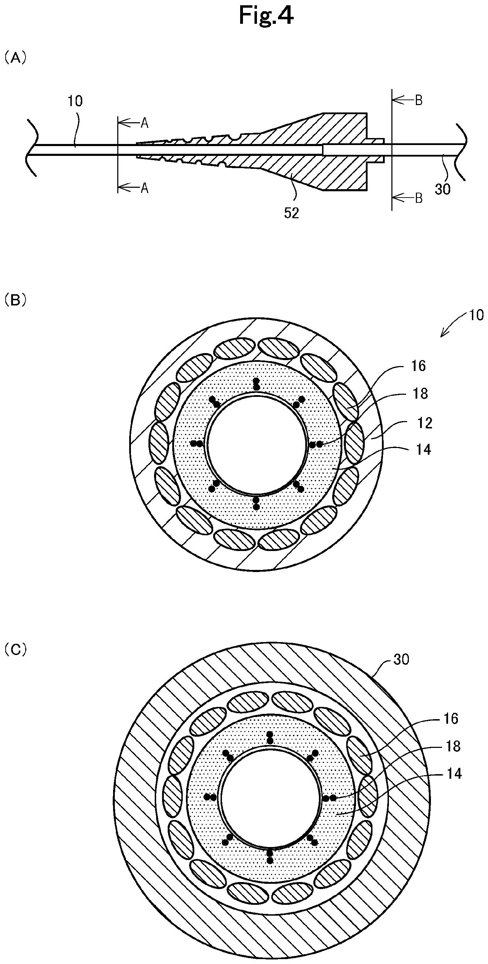

Fig. 4 is an explanatory diagram schematically showing a configuration of the hollow shaft 10 and the proximal end electrode 30. Fig. 4(A) schematically shows the appearance of the hollow shaft 10 and the proximal end electrode 30. Fig. 4(A) illustrates a state in which the distal end grip portion 52 is split along the axis line, and the hollow shaft 10 and the proximal end electrode 30 are exposed. As illustrated, the proximal end electrode 30 is provided on the proximal end portion of the hollow shaft 10.

Fig. 4(B) is an explanatory diagram schematically illustrating a cross-sectional configuration of the hollow shaft 10, and illustrates the A-A cross-section in Fig. 4(A). The hollow shaft 10 includes a hollow outer shaft 12, and a hollow inner shaft 14 disposed inside an outer lumen of the outer shaft 12. The outer shaft 12 and the inner shaft 14 are long, and the transverse cross-sections thereof are substantially circular.

A hollow rope coil 16 formed by winding a wire is embedded inside the outer shaft 12. The wire constituting the rope coil 16 is formed of a conductive metallic material, and may, for example, be formed of stainless steel such as SUS304, nickel-titanium alloy, or an alloy containing gold, platinum, or tungsten, which are X-ray opaque materials. The rope coil 16 is energizably connected to the distal end electrode 20 and the proximal end electrode 30. The outer surface of the outer shaft 12 may be coated with a water-repellent resin or a hydrophobic resin.

A braid 18, which is a reinforcing member formed by braiding a wire, is embedded inside the inner shaft 14. Like the rope coil 16, the wire constituting the braid 18 is formed of a conductive metallic material, and may, for example, be formed of stainless steel such as SUS304, nickel-titanium alloy, or an alloy containing gold, platinum, or tungsten, which are X-ray opaque materials. The rope coil 16 and the braid 18 may be formed of known conductive metallic materials other than those mentioned above.

The outer shaft 12 and the inner shaft 14 are formed of insulating resins, and may be formed of polyolefins such as polyethylene, polypropylene, and ethylenepropylene copolymer, polyesters such as polyethylene terephthalate, thermoplastic resins such as polyvinyl chloride, ethylene-vinyl acetate copolymer, crosslinked ethylene-vinyl acetate copolymer, and polyurethane, polyamide elastomers, polyolefin elastomers, polyurethane elastomers, silicone rubber, or latex rubber. The outer shaft 12 and the inner shaft 14 may be formed of known materials other than those mentioned above.

Fig. 4(C) is an explanatory diagram schematically illustrating a cross-sectional configuration of the proximal end electrode 30, and illustrates the B-B cross-section in Fig. 4(A). The proximal end electrode 30 has a hollow tubular shape, and the transverse cross-section thereof has a substantially circular shape. Because the proximal end electrode 30 has a hollow tubular shape, it is possible to pass the plasma guide wire 400 therethrough, or to inject a chemical solution via the connector 70. The proximal end electrode 30 is formed of a conductive metallic material, and may, for example, be formed of stainless steel such as SUS304, nickel-titanium alloy, aluminum, copper, gold, platinum, tungsten, or an alloy containing at least one of these. The resin layer of the outer shaft 12 in part of the proximal end portion of the hollow shaft 10 is removed to expose the rope coil 16, which is inserted into the lumen of the proximal end electrode 30, and then the proximal end electrode 30 and the rope coil 16 are connected by welding. Like the proximal end electrode 30, the distal end electrode 20 provided on the distal end portion of the hollow shaft 10 is formed of a conductive metallic material, and is connected to the rope coil 16. As a result, the distal end electrode 20 and the proximal end electrode 30 are electrically connected via the rope coil 16. A PTFE (polytetrafluoroethylene) layer may be formed on the inner surface of the proximal end electrode 30. As a result of forming the PTFE layer, the biocompatibility and the slidability can be improved. A PE (polyethylene) layer or a PA (polyamide) layer may be formed instead of the PTFE layer. The PE layer and the PA layer are also capable of improving the biocompatibility and the slidability. Note that, by forming the distal end electrode 20 with an alloy or the like containing gold, platinum, or tungsten, which are X-ray opaque materials, the distal end electrode 20 can also function as an X-ray opaque marker inside a body cavity.

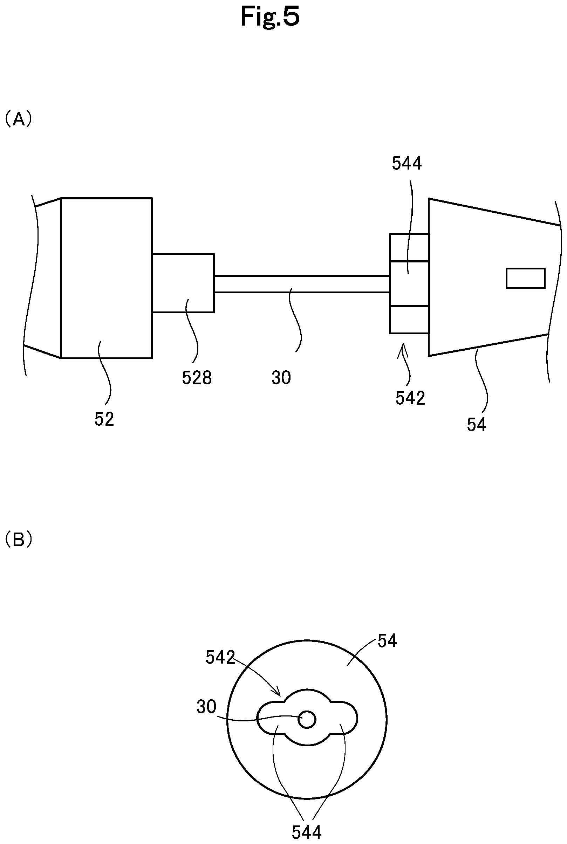

Fig. 5 is an explanatory diagram schematically showing a configuration of a support portion 542 that fixedly supports the proximal end electrode 30. Fig. 5(A) shows a side view, and Fig. 5(B) shows a view from the distal end grip portion 52 side. The proximal end grip portion 54 includes the support portion 542 that fixedly supports the proximal end electrode 30 on the distal end side of the proximal end grip portion 54. The support portion 542 has protrusion portions 544 that protrude from the central axis of the hollow shaft 10 (which coincides with the central axis of the proximal end electrode 30) in directions toward the outer periphery of the intermediate grip portion 56. Furthermore, the distal end grip portion 52 includes, on the proximal end side, a distal end side support portion 528 that fixedly supports the proximal end electrode 30. The distal end side support portion 528 has a cylindrical shape whose central axis coincides with that of the proximal end electrode 30. In the present embodiment, the distal end side support portion 528 is not provided with protrusion portions like the protrusion portions 544, but in another embodiment, the distal end side support portion 528 may be provided with protrusion portions. Moreover, it is possible for the support portion 542 to not be provided with the protrusion portions 544, and for the distal end side support portion 528 to be provided with protrusion portions.

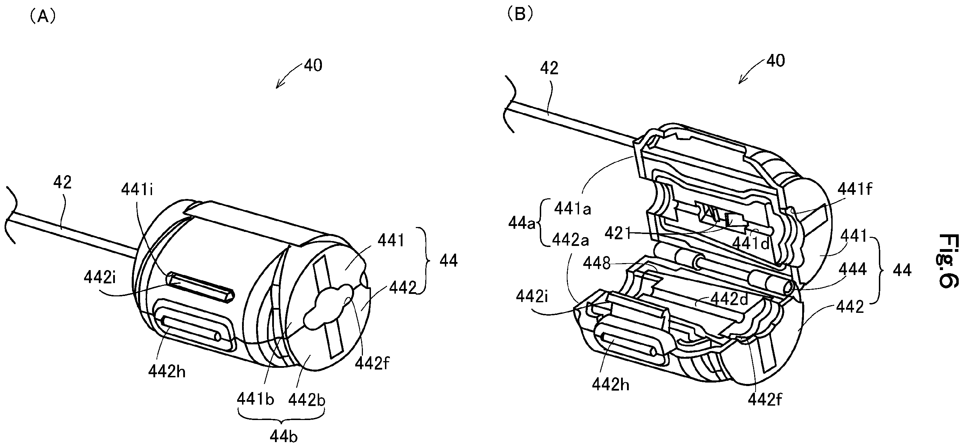

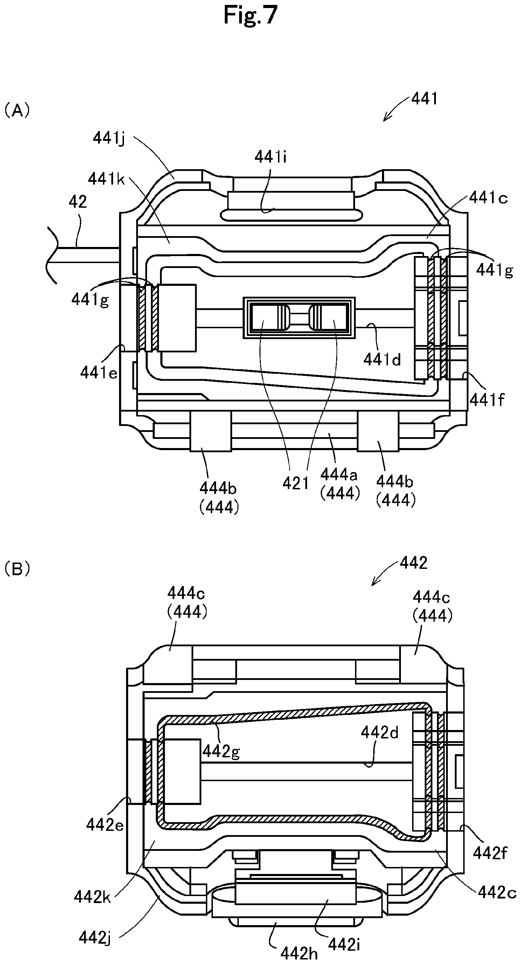

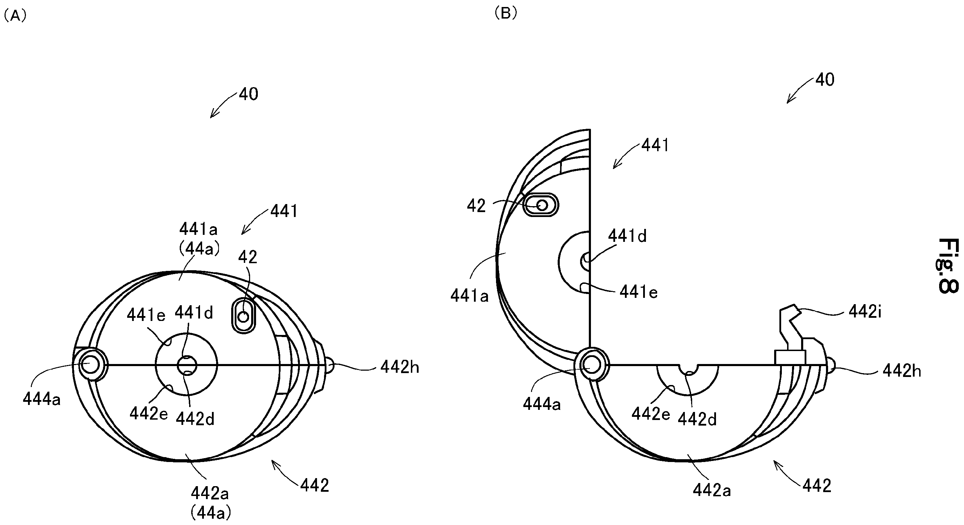

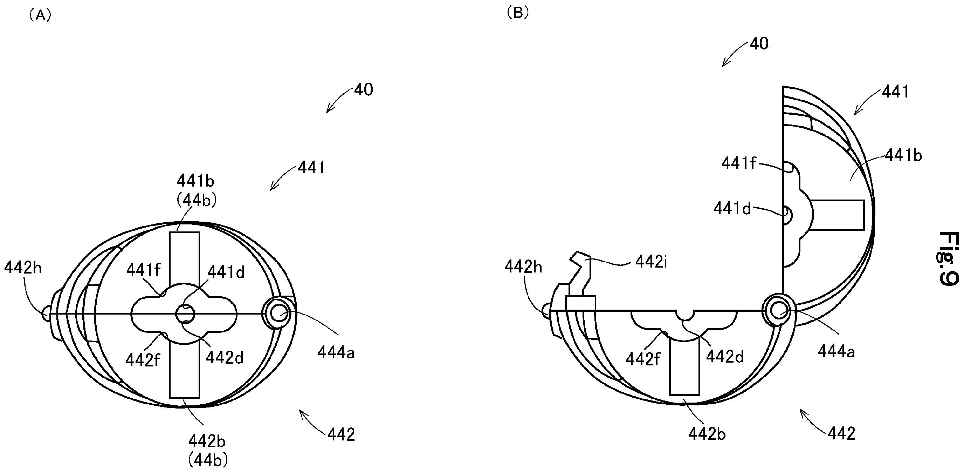

Fig. 6 is an explanatory diagram (perspective view) schematically showing a configuration of the connection portion 40. Fig. 7 is an explanatory diagram schematically showing inner surface configurations of a first cover portion 441 and a second cover portion 442. Fig. 8 is an explanatory diagram schematically showing a distal end surface 44a of the connection portion 40, and Fig. 9 is an explanatory diagram schematically showing a proximal end surface 44b of the connection portion 40. When attached to the proximal end electrode 30, the connection portion 40 has the distal end surface 44a disposed on the distal end side, and the proximal end surface 44b disposed on the proximal end side. Figs. 6(A), 8(A) and 9(A) show a closed state of the connection portion 40, and Figs. 6(B), 8(B) and 9(B) show an open state of the connection portion 40. As mentioned above, the connection portion 40 includes a lead wire 42 and a covering portion 44. The covering portion 44 includes the first cover portion 441 and the second cover portion 442, and the first cover portion 441 and the second cover portion 442 are joined by a joint portion 444 having a hinge structure, and can transition between a closed state (Fig. 6(A)) and an open state (Fig. 6(B)). As shown in Fig. 7, the joint portion 444 includes a shaft 444a, and rotating support portion 444b (Fig. 7(A)) and a rotating support portion 444c (Fig. 7(B)) which have a hole through which the shaft is inserted. The rotating support portion 444b is integrally formed with the first cover portion 441, and the rotating support portion 444c is integrally formed with the second cover portion 442. As a result of the shaft 444a being inserted through the hole of the rotating support portion 444b and the rotating support portion 444c, the first cover portion 441 and the second cover portion 442 are joined so as to be capable of opening and closing. The connection portion 40 is attached to the proximal end electrode 30 in the closed state (Fig. 6(A)), in which the proximal end electrode 30 is held between the first cover portion 441 and the second cover portion 442, and can be detached from the proximal end electrode 30 in the open state (Fig. 6(B)).

As shown in Fig. 7(A), the first cover portion 441 includes an outer shell 441j and an inner shell 441k. Similarly, as shown in Fig. 7(B), the second cover portion 442 includes an outer shell 442j and an inner shell 442k. The outer shell 441j and the outer shell 442j can be made of a relatively rigid material such as polyamide resin, polyethylene resin, ABS (acrylonitrile butadiene styrene) resin, or the like. The inner shell 441k and the inner shell 442k can be made of a relatively flexible material such as silicone resin, elastomer, rubber, or the like.

As shown in Fig. 7, the second cover portion 442 includes a button portion 442h and a hook portion 442i that is connected to the button portion 442h, and the first cover portion 441 includes a hole portion 441i to which the hook portion 442i can be engaged. As shown in Fig. 6(A), when the first cover portion 441 and the second cover portion 442 are in the closed state, the hook portion 442i and the hole portion 441i become engaged and the closed state is maintained. Furthermore, when the button portion 442h is pressed when the first cover portion 441 and the second cover portion 442 are in the closed state, the hook portion 442i that is connected to the button portion 442h is loosened from the hole portion 441i such that the engagement between the hook portion 442i and the hole portion 441i is released, and the first cover portion 441 and the second cover portion 442 adopt the open state. In this way, an operator is capable of attaching the connection portion 40 to the proximal end electrode 30 by causing the first cover portion 441 and the second cover portion 442 to adopt the closed state, and can detach the connection portion 40 from the proximal end electrode 30 by pressing the button portion 442h. That is, according to the connection portion 40 of the present embodiment, it is possible to provide an operator with ease of attachment and detachment.

As shown in Fig. 8, the lead wire 42 is provided so as to penetrate a first distal end surface 441a of the first cover portion 441. A first connection terminal 421, which is disposed at one end of the lead wire 42, is provided so as to be exposed at an inner surface 441c of the first cover portion 441 (Fig. 7(A)). The first connection terminal 421 is disposed in a position making contact with the proximal end electrode 30 when the connection portion 40 is attached to the proximal end electrode 30.

The first cover portion 441 includes, in a section of an inner shell 441k of the inner surface 441c, a groove portion 441d for an electrode in which the proximal end electrode 30 can be fitted. Similarly, the second cover portion 442 includes, in a portion of an inner shell 442k of the inner surface 442c, a groove portion 442d for an electrode in which the proximal end electrode 30 can be fitted. When the first cover portion 441 and the second cover portion 442 are in the closed state, a through hole having a substantially circular shape in transverse cross-section is formed by the groove portion 441d for an electrode and the groove portion 442d for an electrode (Figs. 8(A) and 9(A)). When the connection portion 40 is attached to the proximal end electrode 30, the outer circumferential surface of the proximal end electrode 30 is covered by the groove portion 441d for an electrode and the groove portion 442d for an electrode. As shown in Fig. 7(A), because the first connection terminal 421 is disposed in the groove portion 441d for an electrode, when the proximal end electrode 30 is disposed in the groove portion 441d for an electrode, the proximal end electrode 30 and the first connection terminal 421 are easily disposed in appropriate positions, and are capable of being electrically connected to the RF generator 500 with high accuracy.

The first cover portion 441 includes, in a section of the inner shell 441k of the inner surface 441c, a distal end side concave portion 441e in which the distal end side support portion 528 of the distal end grip portion 52 can be fitted, and a proximal end side concave portion 441f in which the support portion 542 of the proximal end grip portion 54 can be fitted. Similarly, the second cover portion 442 includes, in a portion of the inner shell 442k of the inner surface 442c, a distal end side concave portion 442e in which the distal end side support portion 528 of the distal end grip portion 52 can be fitted, and a proximal end side concave portion 442f in which the support portion 542 of the proximal end grip portion 54 can be fitted. When the first cover portion 441 and the second cover portion 442 are in the closed state, a hole having a lumen shape that substantially matches the shape of the support portion 542 of the proximal end grip portion 54 is formed by the proximal end side concave portion 441f and the proximal end side concave portion 442f (Fig. 9(A)). As described above, because the support portion 542 of the proximal end grip portion 54 includes the protrusion portions 544 that protrude from the central axis of the hollow shaft 10 in directions toward the outer periphery of the grip portion 50, and the connection portion 40 has the proximal end side concave portion 441f and the proximal end side concave portion 442f in which the support portion 542 is fitted, when the connection portion 40 is attached to the proximal end electrode 30 and configures the intermediate grip portion 56, the rotational force applied to the intermediate grip portion 56 when an operator grips the intermediate grip portion 56 and performs a rotation operation of the catheter is transmitted to the proximal end electrode 30 via the support portion 542, which suppresses idle rotation of the intermediate grip portion 56 and enables the torquability of the plasma catheter 100 to be improved. The proximal end side concave portion 441f and the proximal end side concave portion 442f of the present embodiment are also simply referred to as "concave portions".

As shown in Fig. 7(B), in a section of the inner shell 442k of the inner surface 442c of the second cover portion 442, a protrusion-shaped seal portion 442g is formed that surrounds the outer periphery of the groove portion 442d for an electrode. As shown in Fig. 7(A), in a section of the inner shell 441k of the inner surface 441c of the first cover portion 441, a protrusion-shaped seal portion 441g that straddles the distal end side concave portion 441e and a protrusion-shaped seal portion 441g that straddles the proximal end side concave portion 441f are formed so as to correspond to the seal portion 442g of the second cover portion 442. In Fig. 7, the protrusion-shaped seal portions 441g and 442g are illustrated with diagonal hatched lines. When the first cover portion 441 and the second cover portion 442 are in the closed state and the proximal end electrode 30 is connected, the periphery of the proximal end electrode 30 can be surrounded by the seal portions 441g and 442g. Therefore, during use of the plasma catheter 100 and the like, entry of water into the proximal end electrode 30 can be suppressed, which enables electrical leakage to be suppressed. According to the connection portion 40 of the present embodiment, for example, it is possible to provide a waterproof property with respect to wetness that is clinically expected, such as a case where the hand of an operator such as a surgeon is wet with physiological saline. As a result, even when the proximal end electrode 30 becomes wet when attaching and detaching the connection portion 40 during a procedure, because the proximal end electrode 30 is surrounded by the seal portions 442g and 441g as a result of the connection portion 40 being attached, the insulated state can be maintained with the outer shells 441j and 442j of the connection portion 40. Therefore, safety can be provided with respect to electrical leakage during discharge.

Although the replacement portion 60 does not include the lead wire 42 and the first connection terminal 421, it has substantially the same configuration as the connection portion 40, and can be detached from the proximal end electrode 30 by opening and closing the first cover portion and the second cover portion, and a seal portion that surrounds the proximal end electrode 30 is formed on the inner surfaces of the first cover portion and the second cover portion. As a result, even when the replacement portion 60 is attached to the proximal end electrode 30, the proximal end electrode 30 is covered, and is provided with the same waterproof property as when the connection portion 40 is attached. Furthermore, the replacement portion 60 has the same torquability as the connection portion 40.

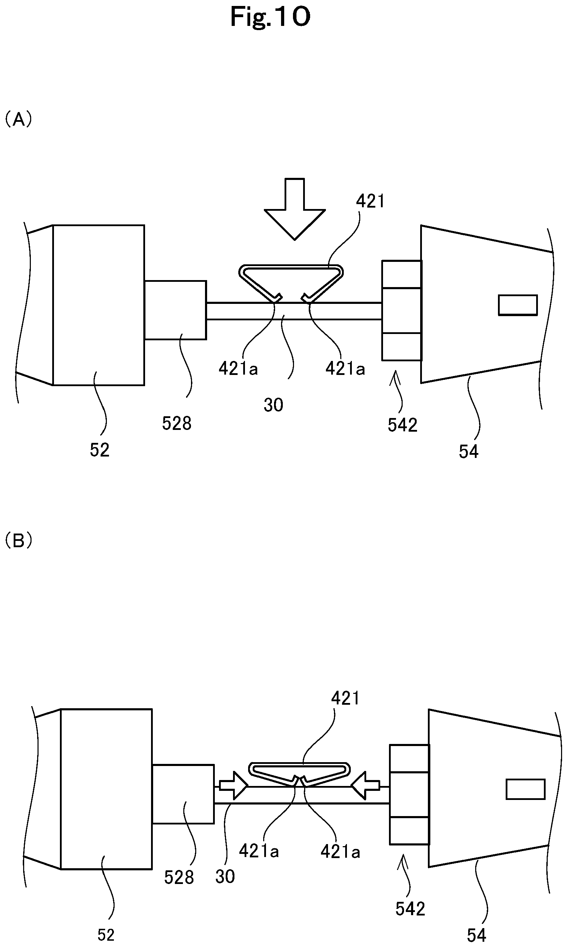

Fig. 10 is an explanatory diagram showing how the first connection terminal 421 makes contact with the proximal end electrode 30. In Fig. 10, in order to clearly show the first connection terminal 421, illustration of the components other than the first connection terminal 421 of the connection portion 40 is omitted. The first connection terminal 421 has two contact portions 421a that make contact with the proximal end electrode 30. When the proximal end electrode 30 is held between the first cover portion 441 and the second cover portion 442, the first connection terminal 421 and the proximal end electrode 30 make contact at the two contact portions 421a (Fig. 10(A)). At this time, the hook portion 442i and the hole portion 441i are not yet engaged. As a result of pressing the first cover portion 441 and the second cover portion 442 from the outside of the first cover portion 441 and the second cover portion 442 toward the inside, the hook portion 442i and the hole portion 441i become engaged and the closed state (locking) of the covering portion 44 is maintained. In this way, when the first cover portion 441 and the second cover portion 442 are pressed, the first connection terminal 421 is flattened, and the two contact portions 421a slide along the surface of the proximal end electrode 30 so as to approach each other. As a result, even when a foreign object adheres to the proximal end electrode 30, it can be removed by the first connection terminal 421, and good electrification can be obtained.

The plasma guide wire system 1 of the present embodiment, for example, is capable of opening a CTO as follows. Initially, an operator uses the plasma catheter 100 in a state where the replacement portion 60 is attached to the proximal end electrode 30 of the plasma catheter 100 and configures the intermediate grip portion 56. First, the proximal end of a delivery guide wire (not illustrated) is inserted from an opening 104a (Fig. 1) at the distal end of the plasma catheter 100, passed through an inner lumen of the inner shaft 14 (Fig. 4), and is made to protrude from the proximal end portion of the inner lumen to the outside. Further, the operator operates the plasma catheter 100 by gripping the grip portion 50 including the replacement portion 60, and delivers the plasma catheter 100 along the delivery guide wire to the CTO that has formed in the coronary artery. Then, the operator injects a contrast medium from the inner lumen of the inner shaft 14 while performing X-ray imaging via the connector 70 (Fig. 1) of the plasma catheter 100, and disposes the distal end portion of the plasma catheter 100 in an optimal position for penetration through to the true lumen. After the plasma catheter 100 is disposed in an optimal position, the operator removes the delivery guide wire and inserts a new plasma guide wire 400 (Fig. 1) into the inner lumen of the inner shaft 14. The operator delivers the distal end section of the plasma guide wire 400 to the distal end portion of the plasma catheter 100, and makes the distal end section of the plasma guide wire 400 protrude from the opening 104a to the outside. Then, the operator detaches the replacement portion 60 of the plasma catheter 100 from the proximal end electrode 30, and instead attaches the connection portion 40 to the proximal end electrode 30. At this time, the lead wire 42 provided in the connection portion 40 is connected to the cable connector 506, and the conduction between the plasma catheter 100 and the RF generator 500 is established as a result of the connection portion 40 being attached to the proximal end electrode 30. The operator causes the RF generator 500 to generate a streamer corona discharge at the distal tip 403 of the plasma guide wire 400 and ablates the CTO.

Note that, for example, after pulling out the delivery guide wire, by inserting an imaging sensor through the inner lumen of the inner shaft 14, position adjustment of the plasma catheter 100 may be performed by an imaging sensor image instead of X-ray imaging.

As described above, because the plasma catheter 100 of the present embodiment includes the connection portion 40, which is configured so as to be capable of being attached to and detached from the proximal end electrode 30, and electrically connects to the RF generator 500 and the proximal end electrode 30 via the lead wire 42 by being attached to the proximal end electrode 30, the plasma catheter 100 can be used such that the connection portion 40 is detached when conduction is not required, and the connection portion 40 is attached when conduction is required. As a result, as described above, when an operator inserts the plasma catheter 100 into the body and performs an operation to advance it inside a blood vessel, good operability that is equivalent to a catheter that does not have a lead wire and the like can be obtained by detaching the connection portion 40 from the proximal end electrode 30, and detaching the lead wire 42 and the first connection terminal 421. On the other hand, when the distal end electrode 20 of the plasma catheter 100 reaches the target position and conduction becomes necessary to perform cauterization or the like, energization can be established by attaching the connection portion 40 to the proximal end electrode 30 and connecting it to the RF generator 500. That is, as a result of providing the connection portion 40 that can be attached and detached, good operability is obtained when conduction is not necessary, and a catheter that can be energized when necessary can be provided.

Furthermore, the plasma catheter 100 is configured such that the connection portion 40 and the replacement portion 60 can be exchanged (Fig. 3(A)). Because the replacement portion 60 does not include the lead wire 42, when the replacement portion 60 is attached to the proximal end electrode 30 and configures the intermediate grip portion 56, a reduction in operability caused by the lead wire becoming entangled with the grip portion 50 and the hollow shaft 10 can be suppressed. Moreover, because the replacement portion 60 is provided with the same seal portion as the connection portion 40, even when the replacement portion 60 is attached, the same waterproof property can be ensured as when the connection portion 40 is attached.

In addition, in the plasma catheter 100, because the connection portion 40 is easily detachable from the proximal end electrode 30 by opening and closing the first cover portion 441 and the second cover portion 442, it is possible to quickly attach the connection portion 40 to the proximal end electrode 30 and quickly provide conduction to the catheter.

### <Second Embodiment>

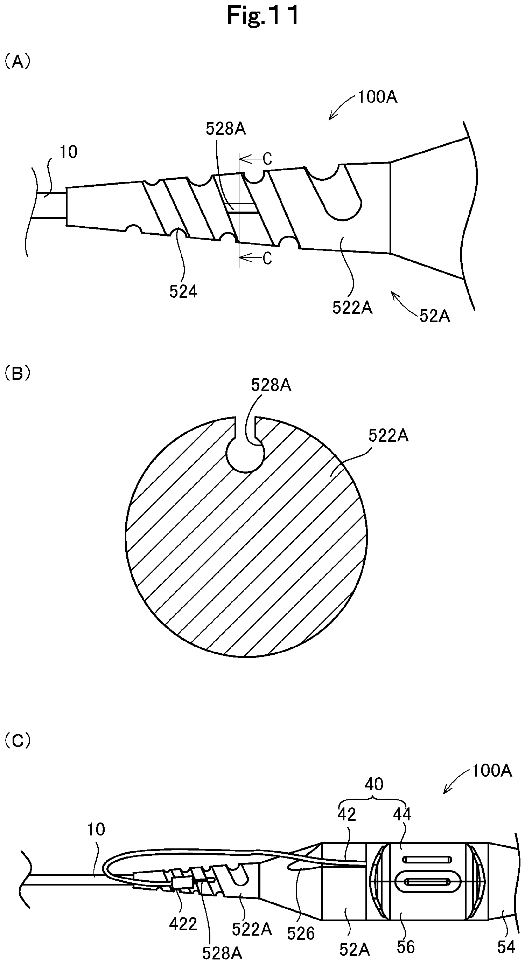

Fig. 11 is an explanatory diagram showing the appearance of a distal end grip portion 52A of a plasma catheter 100A according to a second embodiment. The plasma catheter 100A of the present embodiment differs from the plasma catheter 100 according to the first embodiment in that a terminal support portion 528A that is capable of supporting the second connection terminal 422 of the lead wire 42 is provided. In the embodiment described below, the same components as those of the plasma catheter 100 according to the first embodiment are denoted by the same reference numerals and refer to the preceding description.

As shown in Fig. 11(A), the terminal support portion 528A of the present embodiment is formed in the form of a groove on the surface of a section of a tapered portion 522A of the distal end grip portion 52A in which the spiral groove portion 524 is not formed. Fig. 11(B) illustrates the C-C cross-section in Fig. 11(A), and shows a transverse cross-section of the terminal support portion 528A. As illustrated, at the terminal support portion 528A, the width of the notch section at the surface of the tapered portion 522A has been made narrow such that the distal end part of the second connection terminal 422 does not fall out from the surface side of the tapered portion 522A. In Fig. 11(C), the connection portion 40 is attached to the plasma catheter 100A, and shows how the second connection terminal 422 of the lead wire 42 is supported by the terminal support portion 528A. As a result of an operator inserting the distal end of the second connection terminal 422 from the distal end side of the terminal support portion 528A into the terminal support portion 528A, the second connection terminal 422 can be supported by the distal end side support portion 528.

According to the plasma catheter 100A of the present embodiment, when the connection portion 40 is attached to the proximal end electrode 30, and the second connection terminal 422 is detached from the cable connector 506 (Fig. 1), the electrical connection between the second connection terminal 422 and the voltage output device (RF generator 500) is disconnected, and the plasma catheter 100A is operated, the second connection terminal 422 can be fixed to the distal end grip portion 52A by the terminal support portion 528A. As a result, a reduction in operability caused by the second connection terminal 422 of the lead wire 42 not being fixed and moving freely, and then becoming entangled with the catheter and the like, can be suppressed.

### <Third Embodiment>

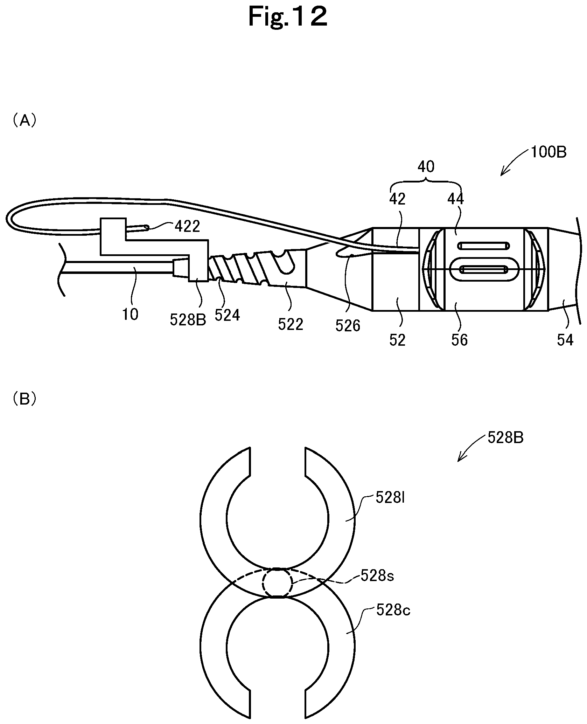

Fig. 12 is an explanatory diagram showing the appearance of a terminal support portion 528B of a plasma catheter 100B according to a third embodiment. The terminal support portion 528B according to the present embodiment differs from the terminal support portion 528A according to the second embodiment, and is configured separately from the distal end grip portion 52. That is, the shape of the distal end grip portion 52 is the same as in the first embodiment. As shown in Fig. 12(A), the terminal support portion 528B of the present embodiment is attached to the distal end side of the tapered portion 522. Fig. 12(B) shows the terminal support portion 528B viewed from the distal end side. As illustrated, the terminal support portion 528B includes a first member 5281 having an open ring shape, a second member 528c having the same open ring shape as the first member 5281, and a third member 528s having a rod shape that connects the first member 528l and the second member 528c. As a result of an operator attaching the terminal support portion 528B to the distal end grip portion 52 by fitting the second member 528c to the tapered portion 522, and by fitting the second connection terminal 422 to the first member 528l, the second connection terminal 422 of the lead wire 42 can be supported by the terminal support portion 528B. In this way, it is possible to suppress a reduction in operability caused by the lead wire in the same manner as in the second embodiment.

### <Fourth Embodiment>

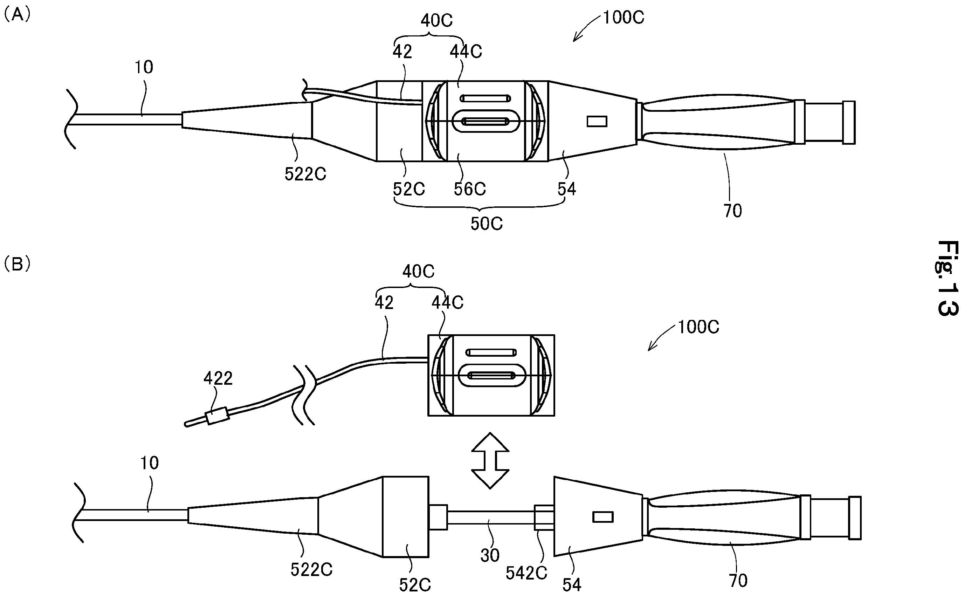

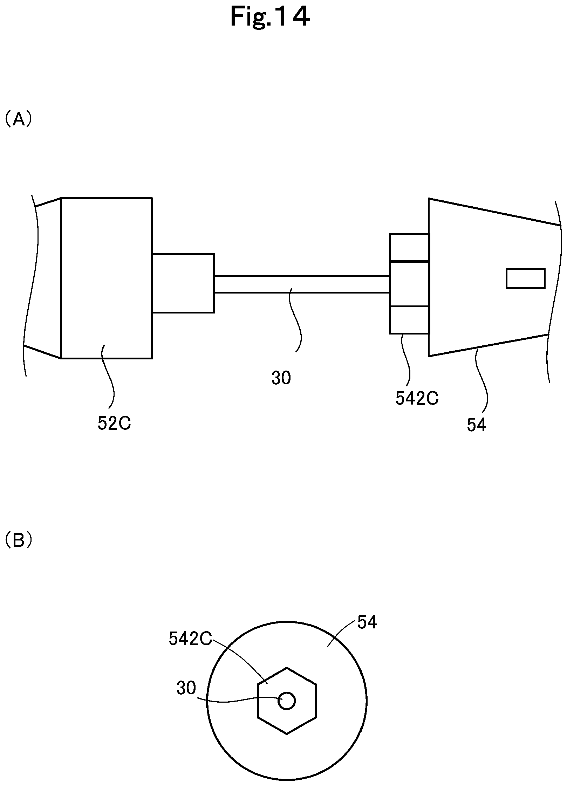

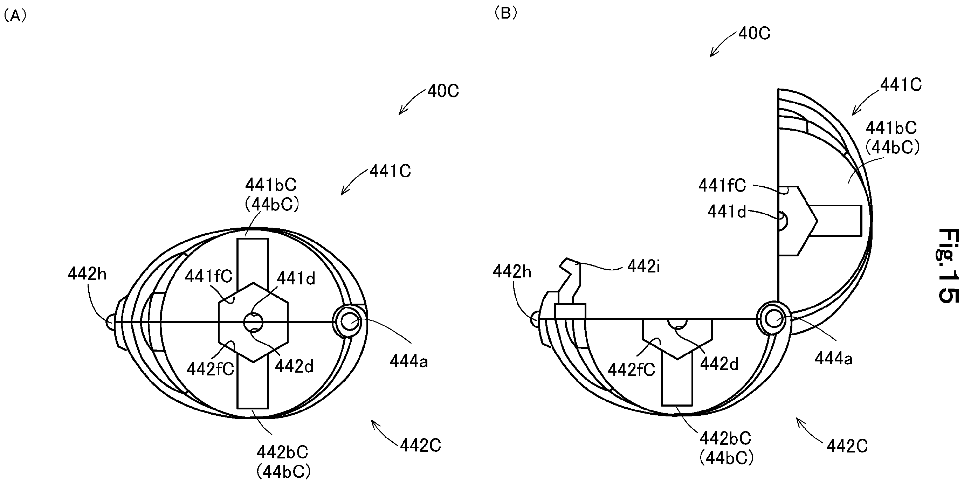

Fig. 13 is an explanatory diagram showing a schematic configuration of a plasma catheter 100C according to a fourth embodiment, Fig. 14 is an explanatory diagram schematically showing a configuration of a support portion 542C that fixedly supports the proximal end electrode 30, and Fig. 15 is an explanatory diagram schematically showing a proximal end surface 44bC of a connection portion 40C. Figs. 13, 14, and 15 respectively correspond to Figs. 2, 5, and 9 of the first embodiment. The plasma catheter 100C of the present embodiment differs from the plasma catheter 100 according to the first embodiment in the shape of the tapered portion 522C of the distal end grip portion 52C, the configuration of the connection portion 40C, and the shape of the support portion 542C of the proximal end grip portion 54.

As shown in Fig. 13, the tapered portion 522C does not include the spiral groove portion 524.

Furthermore, as shown in Fig. 14, the support portion 542C has a substantially hexagonal prism shape, and as shown in Fig. 15, the shape of the transverse cross-section of the hole formed by the proximal end side concave portion 441fC and the proximal end side concave portion 442fC of the connection portion 40C is a substantially hexagonal prism shape that substantially matches the shape of the support portion 542C. Although the support portion 542C is not provided with the protrusion portions 544 that protrude from the central axis of the hollow shaft 10 in directions toward the outer periphery of the grip portion 50, because the support portion 542C has a substantially hexagonal prism shape, and the support portion 542C is fitted to the proximal end side concave portion 441fC and the proximal end side concave portion 442fC of the connection portion 40C, the rotational force applied to the connection portion 40C when an operator grips the connection portion 40C and performs a rotation operation of the plasma catheter 100C is transmitted to the proximal end electrode 30 via the support portion 542C, which suppresses idle rotation of the connection portion 40C and enables a catheter having good torquability to be provided.

### <Fifth Embodiment>

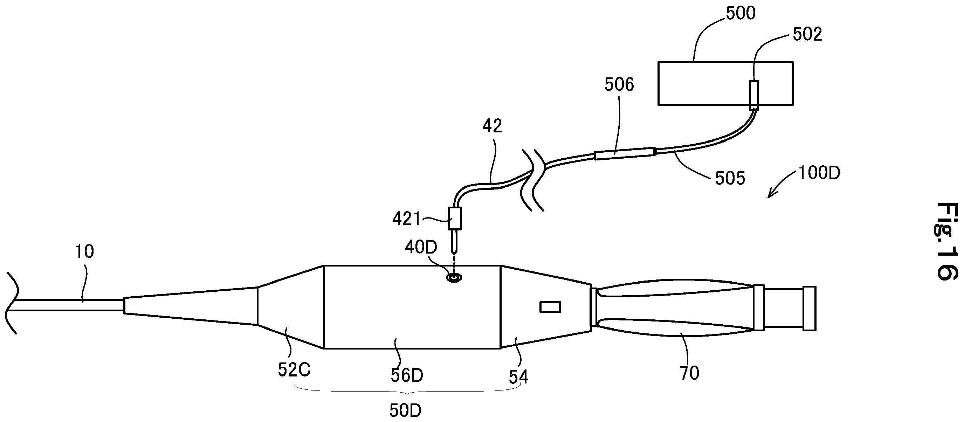

Fig. 16 is an explanatory diagram showing the external configuration of a plasma catheter 100D according to a fifth embodiment. The plasma catheter 100D does not include the lead wire 42, and has a connection portion 40D into which the first connection terminal 421 of the lead wire 42 is inserted. The connection portion 40D is attached to the proximal end electrode 30 as a result of the first connection terminal 421 being inserted, and includes an attachment portion that establishes conduction with the RF generator 500. That is, the connection portion 40D is attached to the proximal end electrode 30 as a result of the first connection terminal 421 being inserted, is detached from the proximal end electrode 30 as a result of the first connection terminal 421 being pulled out, and is attached to and detached from the proximal end electrode 30 in response to the first connection terminal 421 being inserted and pulled out. The connection portion 40D does not cover the proximal end electrode 30. Even in this case, when conduction is required, conduction can be ensured by connecting the lead wire 42, and when conduction is not required, the operability can be improved by detaching the lead wire 42.

### <Modifications of Embodiments>

The disclosed embodiments are not limited to the embodiments described above and may be carried out in various modes without departing from the spirit thereof, and for example, the following modifications are possible.
· In the embodiments described above, the plasma catheter 100 has been illustrated as the catheter, and the plasma guide wire system 1 has been illustrated, but the catheter is not limited to being a plasma catheter. As long the catheter is configured to include a distal end electrode and a proximal end electrode, for example, it may be configured as a catheter for examination or diagnosis. For example, a configuration is possible in which an image sensor is provided.
· In the embodiments described above, a configuration has been illustrated in which the connection portion 40 covers the proximal end electrode 30, but the connection portion 40 does not have to cover the proximal end electrode 30. A configuration is possible in which the proximal end electrode 30 is covered by a different component to the connection portion 40.
· In the embodiments described above, a configuration has been illustrated in which the plasma catheter 100 includes the replacement portion 60, but the replacement portion 60 does not have to be included.
· In the embodiments described above, the proximal end electrode 30 having a hollow tubular shape has been illustrated, but the shape of the proximal end electrode 30 is not limited to that of the embodiments above, and can be formed having various shapes such as a rod shape, an annular shape, or a tip shape. As described in the embodiments above, it is preferable to form the proximal end electrode 30 with a hollow tubular shape and connect it to the hollow shaft 10 because it is possible to insert a guide wire into the inner cavity of the proximal end electrode 30, or inject a chemical solution via the inner cavity of the proximal end electrode 30.
· In the embodiments described above, an example has been illustrated in which the proximal end electrode 30 is electrically connected to the distal end electrode 20 via the rope coil 16 of the hollow shaft 10, but the electrical connection between the proximal end electrode 30 and the distal end electrode 20 is not limited that described in the embodiments above. For example, the connection may be made via the braid 18 of the hollow shaft 10, or a conductive wire for connecting the proximal end electrode 30 and the distal end electrode 20 may be provided in the hollow shaft 10.
· In the embodiments described above, an example has been illustrated in which the connection portion 40 configures the intermediate grip portion 56, but the connection portion 40 does not have to configure the intermediate grip portion 56.

The aspects of the disclosed embodiments have been described above on the basis of the embodiments and modifications, however, the embodiments of the aspects described above are intended to facilitate understanding of the aspects, and are not intended to limit the aspects. The aspects can be modified and improved without departing from the spirit of the aspects and the scope of the claims, and equivalent aspects are included in the aspects. In addition, the aspects can be omitted as appropriate, unless technical features of the aspects are described as essential in the present specification.

### DESCRIPTION OF REFERENCE NUMERALS

1 Plasma guide wire system
10 Hollow shaft
12 Outer shaft
14 Inner shaft
16 Rope coil
18 Braid
20 Distal end electrode
30 Proximal end electrode
40, 40C, 40D Connection portion
42 Lead wire
44 Covering portion
44a Distal end surface
44b, 44bC Proximal end surface
50 Grip portion
52, 52A, 52C Distal end grip portion
54 Proximal end grip portion
56 Intermediate grip portion
60 Replacement portion
70 Connector
100, 100A, 100B, 100C, 100D Plasma catheter
104a Opening
400 Plasma guide wire
403 Distal tip
421 First connection terminal
421a Contact portion
422 Second connection terminal
441 First cover portion
441a First distal end surface
441c Inner surface
441d Groove portion for electrode
441e Distal end side concave portion
441f Proximal end side concave portion
441fC Proximal end side concave portion
441g Seal portion
441i Hole portion
441j Outer shell
441k Inner shell
442 Second cover portion
442c Inner surface
442d Groove portion for electrode
442e Distal end side concave portion
442f, 442fC Proximal end side concave portion
442g Seal portion
442h Button portion
442i Hook portion
442j Outer shell
442k Inner shell
444 Joint portion
444a Shaft
444b, 444c Rotating support portion
500 RF generator
501, 502 Terminal
504, 506 Cable connector
505 Cable
522, 522A, 522C Tapered portion
524 Spiral groove portion
526 Concave portion for lead wire
528 Distal end side support portion
528A, 528B Terminal support portion
528c Second member
528l First member
528s Third member
542, 542C Support portion
544 Protrusion portion

## Claims

1. A catheter comprising:
a hollow shaft;
a distal end electrode provided on a distal end portion of the hollow shaft;
a proximal end electrode provided on a proximal end portion of the hollow shaft, and which is electrically connected to the distal end electrode via the hollow shaft; and
a connection portion which is capable of being attached to and detached from the proximal end electrode and which, by being attached to the proximal end electrode, electrically connects an external voltage output device and the proximal end electrode via a lead wire.

2. The catheter according to claim 1, wherein
the connection portion is provided with a covering portion that covers the proximal end electrode when attached to the proximal end electrode.

3. The catheter according to claim 2, further comprising
a grip portion provided on a proximal end portion of the hollow shaft, the grip portion being provided with a distal end grip portion disposed on a distal end portion of the grip portion, a proximal end grip portion disposed on a proximal end portion of the grip portion, and an intermediate grip portion disposed between the distal end grip portion and the proximal end grip portion; wherein
the connection portion configures the intermediate grip portion.

4. The catheter according to claim 3, further comprising
a replacement portion that is capable of being attached to and detached from the proximal end electrode, and when the connection portion is not attached to the proximal end electrode and the replacement portion is attached to the proximal end electrode instead of the connection portion, configures the intermediate grip portion that does not electrically connect the proximal end electrode and the voltage output device.

5. The catheter according to any one of claims 2 to 4, wherein
the covering portion includes a first cover portion having a first connection terminal that is connected to the lead wire, and a second cover portion joined with the first cover portion by a joint portion having a hinge structure, is configured to be capable of opening and closing, is attached to the proximal end electrode in a closed state in which the proximal end electrode is held between the first cover portion and the second cover portion, and is capable of being detached from the proximal end electrode in an open state, and
the first connection terminal is disposed in a position making contact with the proximal end electrode when the connection portion is attached to the proximal end electrode.

6. The catheter according to claim 5, wherein
at least one of the first cover portion and the second cover portion is provided with a groove portion for an electrode in which the proximal end electrode can be fitted, and
the groove portion for an electrode is disposed in a position in which the first connection terminal makes contact with the proximal end electrode when the connection portion is attached to the proximal end electrode.

7. The catheter according to claim 6, wherein
at least one of the first cover portion and the second cover portion is provided with a seal portion that surrounds an outer periphery of the groove portion for an electrode.

8. The catheter according to any one of claims 5 to 7, wherein
the proximal end grip portion is provided with a support portion that is provided on a distal end side of the proximal end grip portion and fixedly supports the proximal end electrode,
the support portion has a protrusion portion that protrudes from a central axis of the hollow shaft toward a direction of an outer periphery of the grip portion, and
the intermediate grip portion is provided with a concave portion that is fitted with the support portion of the proximal end grip portion.

9. The catheter according to any one of claims 3 to 8, wherein
the distal end grip portion is provided with a tapered portion having a diameter that decreases toward a distal end thereof, and
the tapered portion has, on a surface, a spiral groove portion formed having a spiral groove shape.

10. The catheter according to any one of claims 3 to 9, wherein
the distal end grip portion is capable of supporting the lead wire along a surface of the distal end grip portion when the intermediate grip portion is configured by the connection portion, and has, on a surface thereof, a concave portion for a lead wire in which a lead wire can be fitted.

11. The catheter according to any one of claims 3 to 10, wherein
the lead wire is provided with a second connection terminal that is disposed on a distal end thereof and is capable of being electrically connected to the voltage output device, and
the catheter is provided with a terminal support portion on the distal end grip portion that is capable of supporting the second connection terminal.
